# EUROPEAN PATENT SPECIFICATION

(11) **EP 3 954 332 B1**
(45) Date of publication and mention of the grant of the patent: **26.07.2023**
(21) Application number: 21189849.9
(22) Date of filing: 05.08.2021
(51) Int. Cl.: A61F 2/30, A61F 2/34

(54) **ACETABULAR CUP ASSEMBLY FOR HIP PROSTHESIS**
AZETABULUMPFANNE FÜR HÜFTPROTHESE
ENSEMBLE CUPULE ACÉTABULAIRE POUR PROTHÈSE DE HANCHE

(30) Priority: 13.08.2020 IT 202000020107
(43) Date of publication of application: 16.02.2022
(73) Proprietor: Melozzi, Alessandro, 64100 Teramo (TE) (IT)
(72) Inventor: Melozzi, Alessandro, 64100 Teramo (TE) (IT)
(74) Representative: Cutropia, Gianluigi

(56) References cited:
- FR-A1- 2 786 390
- KR-A- 20130 090 045
- US-A1- 2009 259 318
- US-B1- 6 537 321
- US-B2- 7 713 306

## Description

The present invention relates to an acetabular cup assembly for hip prosthesis.

As it is known, a hip prosthesis is used to reconstruct the head of a femur of a patient. Therefore, such a hip prosthesis comprises a stem suitable for being disposed in the femur of the patient, an acetabular cup suitable for being engaged in an acetabular seat of the patient, and a spherical head fixed to the stem and suitable for being engaged in the acetabular cup in spherical coupling mode in such a way to form an omnidirectional spherical joint.

In a double-mobility prosthesis, an insert shaped like a segment of a sphere is disposed between the spherical head and the acetabular cup.

The acetabular cups of the prior art are impaired by the fact that they are not versatile and are difficult to adjust to the shape of the acetabular seat of the patient. For this reason, the surgeon needs to be provided with various types of acetabular cups in such a way to choose the most suitable one the shape of the acetabular seat of the patient.

In fact, different types of acetabular cups are to be provided in case of anteversion and valgus condition of the acetabular seat of the patient.

Moreover, the fixing of the acetabular cups of the prior art to the acetabular seat is complicated, inefficient and unsafe.

US7713306B2 discloses a hip prosthesis that comprises a plate shaped like a perforated cup and perforated wings that protrude radially from the cup. The plate is fixed to the acetabular seat and a cup-shaped insert is fixed to the perforated cup of the plate. When the insert is fixed to the plate, the insert cannot slide with respect to the plate. A prosthesis head is disposed inside the insert to slide with respect to the insert. The cup-shaped insert is fixed to the plate, which acts as reinforcement for the insert, and not to the acetabular seat. The perforated cup of the plate is used to reinforce and hold the insert, and not to generate a seat wherein the head of the femur can slide. The plate does not hold the head of the prosthesis.

US2009259318A1 discloses an acetabular assembly made of two parts, comprising an acetabular cup fixed to the acetabular seat and a cup-shaped insert eccentrically disposed in the acetabular cup. The hip prosthesis comprises a head disposed inside the insert.

US6537321B1 discloses an acetabular assembly made of two parts, comprising an acetabular cup fixed to the acetabular seat and an insert fixed to the acetabular cup. The hip prosthesis comprises a head eccentrically disposed inside the insert.

The purpose of the present invention is to eliminate the drawbacks of the prior art by disclosing an acetabular cup assembly for hip prosthesis that is versatile and capable of adjusting to the configuration of the acetabular seat of the patient wherein is installed.

Another purpose of the present invention is to disclose such an acetabular cup assembly that can be fixed o the acetabular seat in a safe, reliable and simple way during surgery.

These purposes are achieved according to the invention with the characteristics of the independent claim 1.

Advantageous embodiments of the invention appear from the dependent claims.

Additional features of the invention will be clearer from the following detailed description, which refers to a merely illustrative, not limiting embodiment, as shown in the appended figures, wherein:
Fig. 1 is an exploded perspective view of the various parts of the acetabular cup assembly according to the invention applied to a hip prosthesis, wherein the stem is shown in a diagrammatic way;
Fig. 2 is a bottom view of the acetabular cup of Fig. 1;
Fig. 2A is a geometric view like Fig. 2;
Fig. 3 is a side view of the acetabular cup of Fig. 1;
Figs. 4 and 5 are two perspective views taken from different angles, which show a growth support coupled with the acetabular cup;
Fig. 6 is a bottom view of a fixing plate applied to the acetabular cup;
Fig. 7 is a view that shows an acetabular seat of the patient prepared to accommodate the acetabular cup assembly according to the invention;
Figs. 8 and 9 are perspective views taken from different angles, which show the acetabular cup assembly disposed in the acetabular seat of the patient and a head mounted in the acetabular cup assembly; and
Fig. 10 shows four possible positions of the acetabular cup according to the type of problem in the hip of the patient.

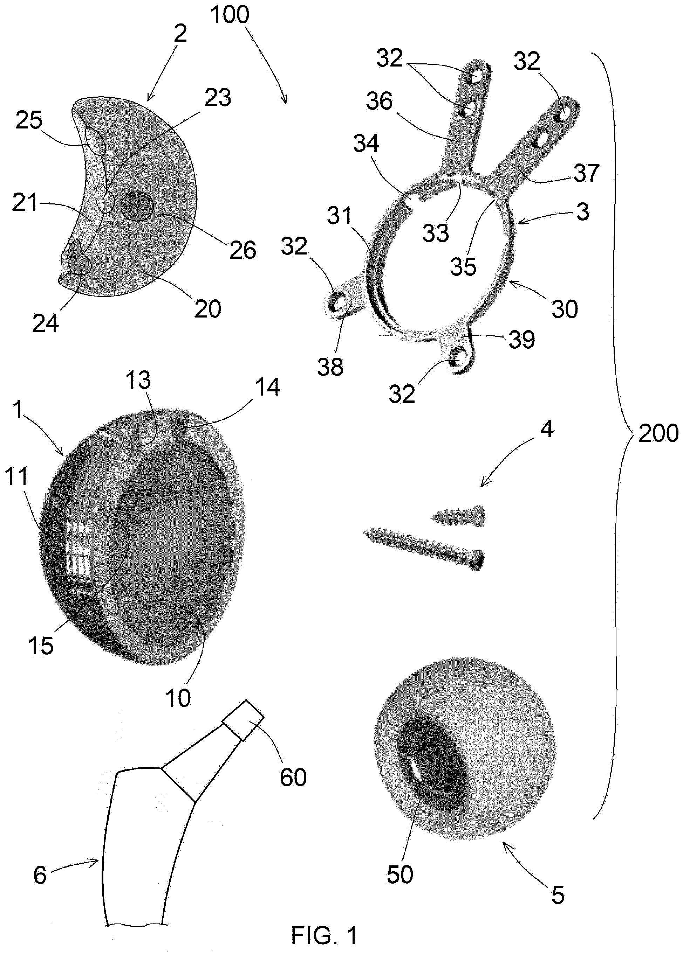

Fig. 1 shows a hip prosthesis (200) comprising an acetabular cup assembly (100) according to the invention.

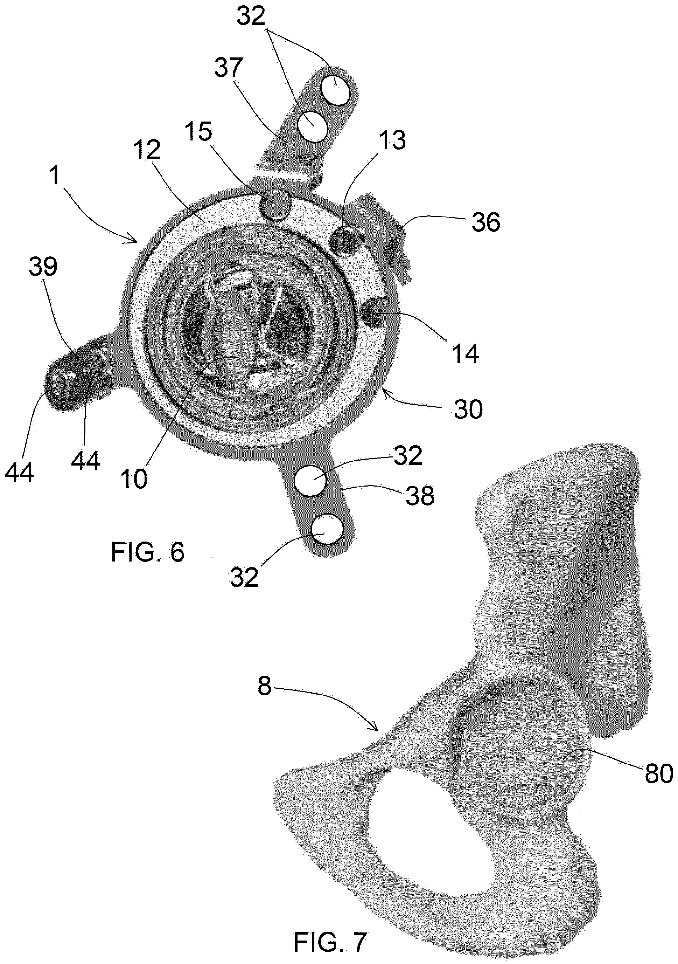

The hip prosthesis (200) comprises:
- a stem (6) suitable for being fixed in a femoral channel of the patient,
- a head (5) with a substantially spherical shape, fixed to the stem (6), and
- the acetabular cup assembly (100) suitable for being applied in a seat (80) (Fig. 7) of an acetabulum (8) of the patient.

The acetabular cup assembly (100) comprises:
- an acetabular cup (1) suitable for being fixed in the seat (80) of the acetabulum (8) of the patient,
- a growth support (2) suitable for being fixed to the acetabular cup (1) and to the acetabular seat (80),
- a fixing plate (3) suitable for being fixed to the acetabulum (8) of the patient in order to hold the acetabular cup (1), and
- screws (4) for fixing the acetabular cup, the growth support (2) and the fixing plate (3) to the acetabular bone.

The acetabular cup (1) is directly coupled in the acetabular seat (80) in press-fit coupling mode.

The fixing plate (3) is a separate piece from to the acetabular cup (1) and is configured in such a way to be fixed to the acetabulum (8) and hold the acetabular cup (1) and the head (5) of the prosthesis.

The head (5) of the hip prosthesis is coupled with the acetabular cup (1) in spherical coupling mode, forming an omnidirectional spherical joint. It must be noted that the head (5) of the hip prosthesis is directly coupled with the acetabular cup (1), without the interposition of any insert between acetabular cup and prosthetic head.

The stem (6) has an upper ending portion (60) with truncated conical shape that is coupled in a truncated conical seat (50) of the head in Morse taper coupling mode.

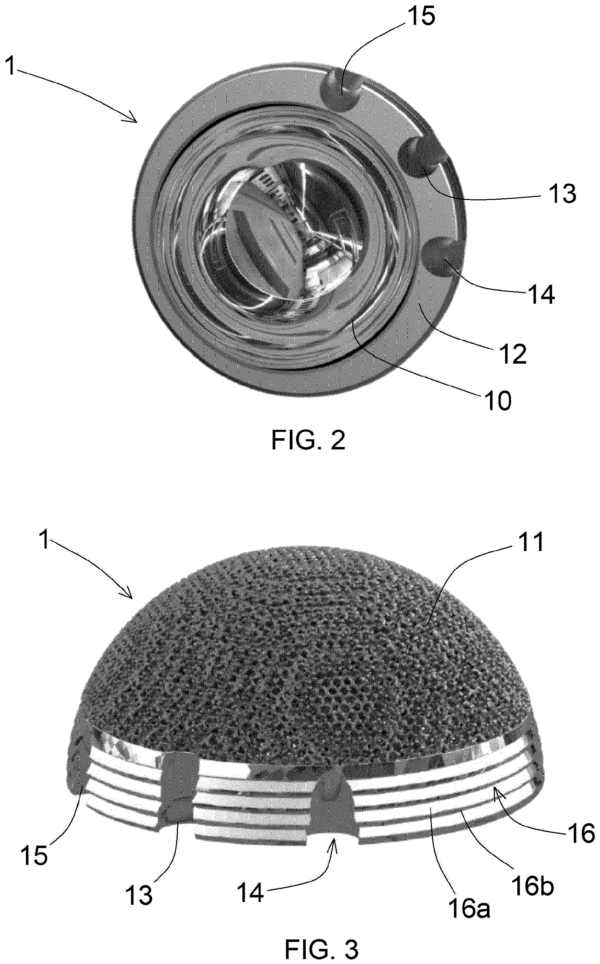

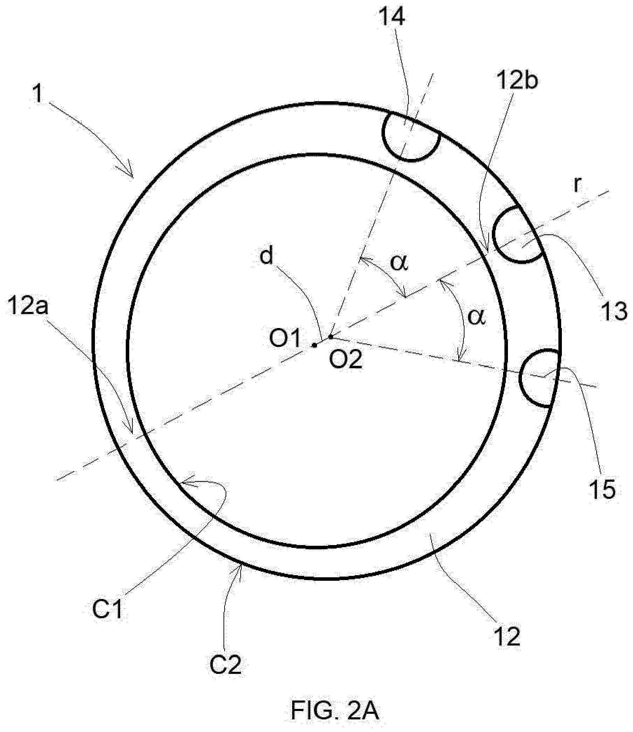

With reference to Figs. 2 and 3, the acetabular cup (1) is shaped as a segment of a sphere with a concave internal surface (10) suitable for accommodating the head (5), and a convex external surface (11) suitable for going in contact with the bone of the patient in the acetabular seat (80).

The acetabular cup (1) has a lower edge (12) with a flat annular shape.

With reference to Fig. 2A, the lower edge (12) of the acetabular cup is defined by an internal circumference (C1) and by an external circumference (C2).

The internal circumference (C1) is eccentric with respect to the external circumference (C2). Otherwise said, the internal circumference (C1) has a center (O1) spaced from a center (O2) of the external circumference (C2) by a distance (d) of approximately 1-5 mm.

Consequently, the lower edge (12) of the acetabular cup has a portion with minimum thickness (12a) diametrically opposite to a portion with maximum thickness (12b) relative to a straight line (r) passing through the centers (O1, O2) of the internal circumference and of the external circumference.

An intermediate hole (13) is obtained peripherally in the portion with maximum thickness (12b) of the lower edge. The intermediate hole (13) is disposed between two lateral holes (14, 15) obtained in peripheral position in the lower edge (12) of the acetabular cup. Each lateral hole (14, 15) is angularly spaced from the intermediate hole (13) by an angle (α) of approximately 25-35°, preferably 30°.

With reference to Fig. 3, the intermediate hole (13) and the lateral holes (14) have axes substantially orthogonal to the lower edge (12) and are open laterally on the convex external surface (11), extending for a height that is approximately 1/5 - 1/2 of the height of the acetabular cup. The holes (13, 14, 15) of the acetabular cup are used to provide a primary fixing of the acetabular cup to the bone of the cotyloid cavity of the patient, when pressure-fixing is not optimal, and/or are used to connect the growth support (2) or the fixing plate (3) in case of revision of the prosthesis.

The convex external surface (11) of the acetabular cup has a fixing portion (16) that extends from the lower edge (12) for a height that is approximately 1/5 - 1/2 of the height of the acetabular cup. The fixing portion (16) has a double function:
- to provide a press-fit coupling with the acetabular seat (80);
- to hold the fixing plate (3) in position.

Advantageously, the fixing portion (16) of the acetabular cup is provided with a plurality of circumferential ribs (16a) and circumferential grooves (16b) between the ribs. The circumferential ribs (16a) have a tapered surface with decreasing dimensions going upwards, in such a way to favor the insertion of the acetabular cup (1) in the acetabular seat.

Advantageously, the circumferential ribs (16a) extend like a helical thread that provides for screwing the acetabular cup in the bone of the cotyloid cavity of the patient.

The acetabular cup (1) is made of a metallic material, such as cobalt-chrome with nitride titanium coating for allergic patients. The convex external surface (11) of the acetabular cup has a trabecular structure, for example of trabecular cobalt-chrome added with hydroxyapatite, in order to provide osteointegration with the bone of the acetabulum of the patient. The fixing portion (16) does not have a trabecular structure.

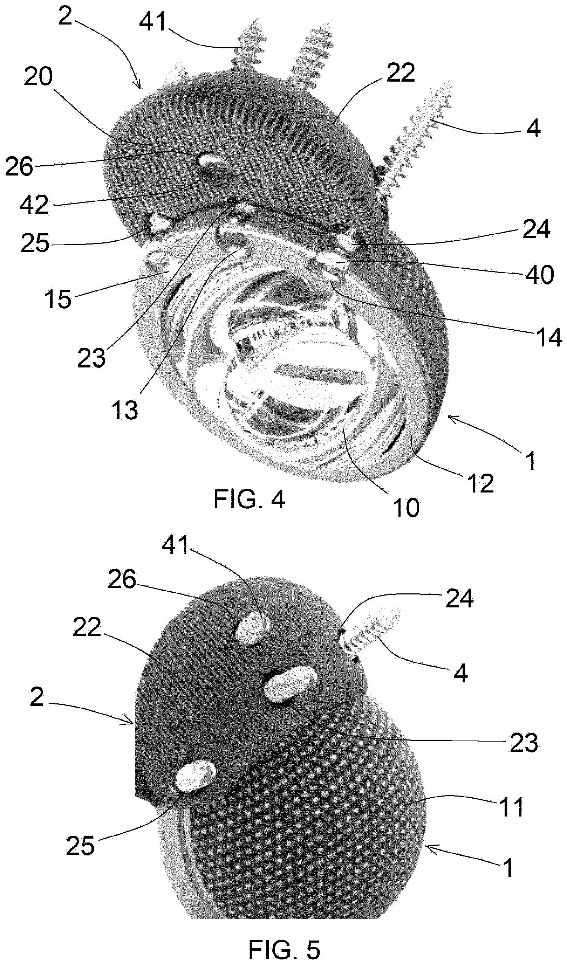

With reference to Figs. 4 and 5, the growth support (2) has a flat lower surface (20) shaped as a moons' quarter, a concave internal surface (21) and a convex external surface (22).

The concave internal surface (21) of the growth support (2) has a section shaped like an arc of circumference and is suitable for being coupled with a portion of the convex external surface (11) of the acetabular cup for an arc of circle subtended by an angle of approximately 50-80°.

In such a way, the growth support (2) protrudes laterally from the acetabular cup, increasing the dimensions of the acetabular cup.

Four holes (23, 24, 25), namely an intermediate hole (23), two lateral holes (24, 25) and a central hole (26), are provided in the flat lower surface (20) of the growth support.

The intermediate hole (23) is disposed between the two lateral holes (24, 25). The intermediate hole (23) and the two lateral holes (24, 25) are disposed in the boundary with the concave internal surface (21), and have axes substantially orthogonal to the flat lower surface (20) of the growth support. The lateral holes (24, 25) are angularly spaced from the intermediate hole (23) by an angle of approximately 25-35°, preferably 30°. In view of the above, when the growth support (2) is mounted on the acetabular cup (1), the intermediate hole (23) and the two lateral holes (24, 25) of the growth support exactly correspond with the intermediate hole (13) and with the two lateral holes (14, 15) of the acetabular cup.

Screws (4) are inserted in the intermediate and lateral holes (13, 14, 15) of the acetabular cup and in the intermediate and lateral holes (23, 24, 25) of the growth support and are screwed in the bone of the acetabulum of the patient, in such a way to firmly fix the acetabular cup (1) and the growth support (2) to the bone of the acetabulum of the patient.

A head (40) of the screws (4) is housed in the intermediate and lateral holes (13, 14, 15) of the acetabular cup. For such a purpose, the intermediate and lateral holes (13, 14, 15) of the acetabular cup have an enlarged seat in order to accommodate the head (40) of the screws.

The central hole (26) is disposed in a substantially central position of the flat lower surface (20) of the growth support and has an axis orthogonal to the flat lower surface (20).

A screw (41) is inserted in the central hole (26) of the growth support and is screwed in the bone of the acetabulum of the patient, in such a way to firmly fix the growth support (2) to the bone of the acetabulum of the patient.

A head (42) of the screw (41) is housed in the central hole (26) of the growth support. For such a purpose, the central hole (26) of the growth support has an enlarged seat in order to accommodate the head (42) of the screw (41).

The growth support (2) is made of trabecular titanium. The flat lower surface (22) and the convex external surface (22) have a trabecular structure, for example of trabecular titanium or hydroxyapatite.

The fixing plate (3) has a collar (30) shaped like a circumference with internal diameter equal to the external diameter of the external circumference (C2) of the flat lower hole (12) of the acetabular cup. In such a way, the collar (30) of the fixing plate can be disposed around the flat lower edge (12) of the acetabular cup and can be screwed in the ribs (16a) shaped like a thread of the acetabular cup.

The fixing plate (30) has a step (31) directed inwards, which is obtained from a circumference with a lower internal diameter. Such a step (31) is used to hold the fixing plate in the rib (16a) shaped like a tread of the acetabular cup.

The fixing plate (3) is provided with an intermediate slot (33) and with two lateral slots (34, 25) that are suitable for being disposed in correspondence with the intermediate hole (13) and with the lateral holes (14, 15) of the acetabular cup.

A first deformable bracket (36) and a second deformable bracket (37) protrude radially outwards from the collar (30) of the fixing plate in correspondence with the central slot (33) and with a lateral slot (35).

The first and second bracket (36, 37) are shaped like an elongated plate and are angularly spaced by an angle of approximately 25-35°.

A third deformable bracket (38) and a fourth deformable bracket (39) protrude radially outwards from the collar (30) of the fixing plate.

The third and fourth bracket (38, 39) are shaped like an elongated plate with a shorter length than the first and second bracket. The third and fourth bracket (38, 39) are angularly spaced by an angle of approximately 60-100°.

The third bracket (38) is angularly spaced from the first bracket (36) by an angle of approximately 90-130°. The fourth bracket (39) is angularly spaced from the second bracket (37) by an angle of approximately 90-130°.

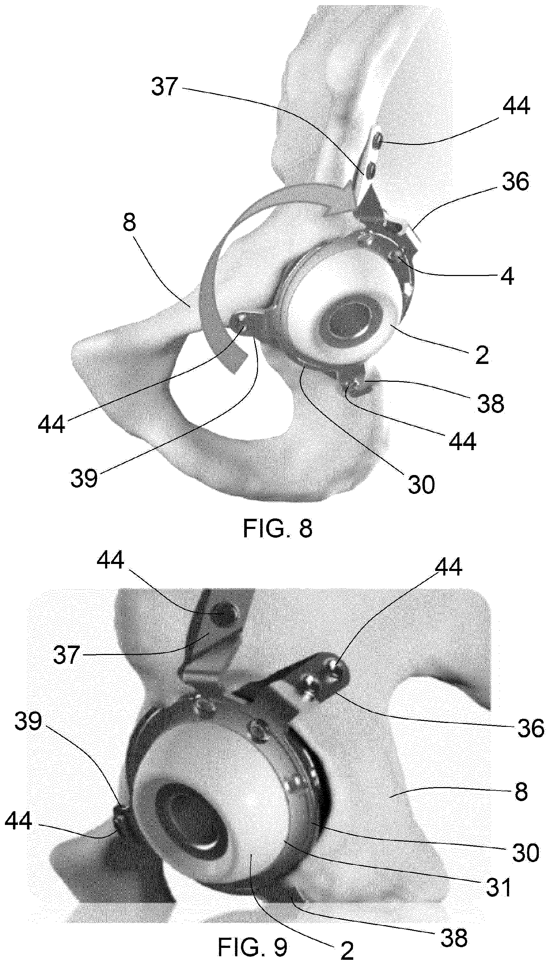

Two holes (32) are provided in the first and second bracket (36, 37). Only one hole (32) is provided in the third and fourth bracket (38, 39). With reference to Figs. 8 and 9, the holes (32) of the brackets are suitable for accommodating screws (44) that are screwed in the bone of the acetabulum (8) in such a way to firmly fix the fixing plate (3) to the acetabulum (8). The screws (44) have a head with higher diameter than the diameter of the holes (32) of the brackets of the fixing plate, in such a way to hold the brackets of the fixing plate.

Fig. 6 shows a fixing plate, wherein the third and fourth bracket (38, 39) are provided with two holes (32) in order to accommodate two screws (44).

The fixing plate (3) is made of a metallic material, such as steel, nitride coated steel, or titanium.

The first and second bracket (36, 37) of the fixing plate have a thickness of approximately 1-3 mm in such a way to be deformed and modelled according to the shape of the acetabulum of the patient, as shown in Figs. 8 and 9.

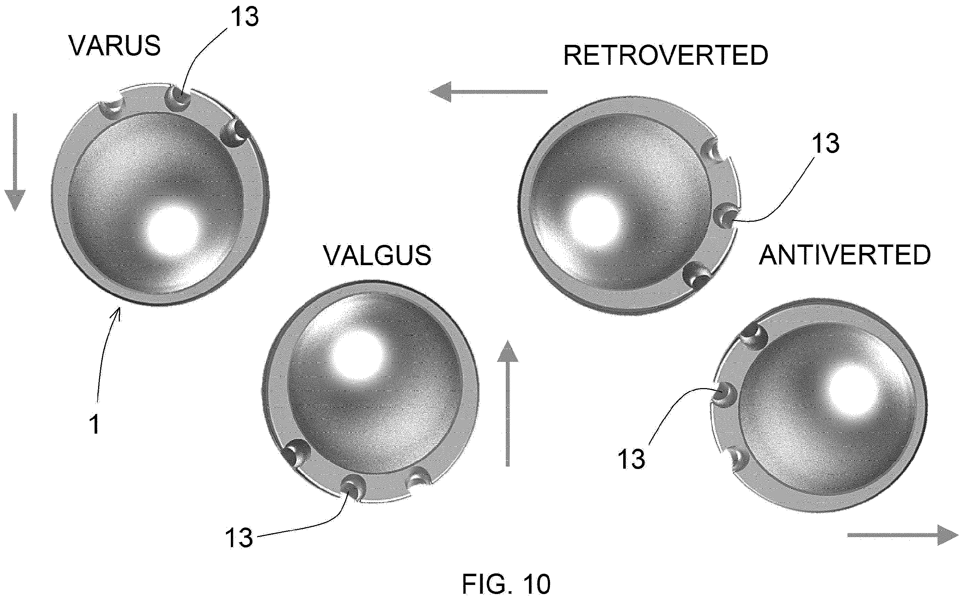

As shown in Fig. 10, it is simply necessary to rotate the acetabular cup (1) in order to change the direction of the femur of the patient. In view of the above, hip problems, such as anteversion and valgus condition, can be corrected. In particular, Fig. 10 shows four possible positions of the acetabular cup (1) according to the type of problem in the hip of the patient: varus, valgus, retroverted, or anteverted hip.

## Claims

1. Acetabular cup assembly (100) comprising:
- an acetabular cup (1) suitable for being fixed in a seat (80) of an acetabulum (8) of the patient and for accommodating a head (5) with a substantially spherical shape fixed to a stem (6) suitable for being disposed in a femur of the patient,
- a growth support (2) suitable for being fixed to the acetabular cup (1) and to the acetabular seat (80),
- a fixing plate (3) suitable for being fixed to the acetabulum (8) of the patient, and
- screws (4) for fixing the acetabular cup, the growth support (2), and the fixing plate (3) to the acetabular bone,
wherein
the acetabular cup (1) is suitably configured for being coupled in press-fit coupling mode to the acetabular seat (80),
**characterized in that**
the fixing plate (3) is a separate element from to the acetabular cup (1) that is suitable to be fixed to the acetabulum (8) in order to hold the acetabular cup (1) and the head (5) of the prosthesis.

2. The acetabular cup assembly (100) of claim 1, wherein the acetabular cup (1) is shaped like a segment of a sphere comprising:
- a concave internal surface (10) suitable for accommodating the head (5) of the prosthesis,
- a convex external surface (11) suitable for going in contact with the bone of the patient in the acetabular seat (80), and
- a lower edge (12) with a flat annular shape, wherein holes (13, 14, 15) are obtained to accommodate screws (4) for fixing the acetabular cup to the acetabulum.

3. The acetabular cup assembly (100) of claim 2, wherein the lower edge (12) of the acetabular cup is defined by an internal circumference (C1) and by an external circumference (C2); the internal circumference (C1) being eccentric with respect to the external circumference (C2); the internal circumference (C1) having a center (O1) spaced from a center (O2) of the external circumference (C2); the lower edge (12) of the acetabular cup having a portion with minimum thickness (12a) diametrically opposite to a portion with maximum thickness (12b), relative to a straight line (r) passing through the centers (O1, O2) of the internal circumference and of the external circumference.

4. The acetabular cup assembly (100) of claim 3, wherein said holes of the acetabular cup comprise an intermediate hole (13) obtained in peripheral position in the portion with maximum thickness (12b) of the lower edge, between two lateral holes (14, 15) obtained in peripheral position in the lower edge (12) of the acetabular cup.

5. The acetabular cup assembly (100) of claim 4, wherein each lateral hole (14, 15) is angularly spaced from the intermediate hole (12) by an angle (α) of approximately 25-35°.

6. The acetabular cup assembly (100) of claim 4 or 5, wherein the intermediate hole (13) and the lateral holes (14) have axes substantially orthogonal to the lower edge (12) and are open laterally on the convex external surface (11), extending for a height that is approximately 1/5 - 1/2 of the height of the acetabular cup.

7. The acetabular cup assembly (100) of any one of claims 2 to 6, wherein the convex external surface (11) of the acetabular cup has a fixing portion (16) that extends from the lower edge (12) for a height that is approximately 1/5 - 1/2 of the height of the acetabular cup; said fixing portion (16) being suitable for providing a press-fit coupling with the acetabular seat (80) and for holding the fixing plate (3) in position.

8. The acetabular cup assembly (100) of claim 7, wherein the fixing portion (16) of the acetabular cup is provided with a plurality of circumferential ribs (16a) and of circumferential grooves (16b) between the ribs; the circumferential ribs (16a) being configured like a helical thread and having a tapered surface with decreasing dimensions going upwards, in such a way to favor the insertion of the acetabular cup (1) in the acetabular seat (80).

9. The acetabular cup assembly (100) according to any one of claims 2 to 8, wherein said growth support (2) comprises:
- a concave internal surface (21) with a section shaped like an arc of circumference suitable for being coupled with a portion of the convex external surface (11) of the acetabular cup in such a way that the growth support (2) protrudes laterally from the acetabular cup,
- a convex external surface (22), and
- a flat lower surface (20) shaped as a moons' quarter, wherein peripheral holes (23, 24, 25) are obtained in such a way to be disposed in correspondence with said holes (13,14, 15) of the acetabular cup in order to accommodate screws (40) that are inserted through the holes of the acetabular cup and of the growth support and are screwed in the acetabulum;
said growth support (2) comprising a central hole (26) suitable for accommodating a screw (41) suitable for being screwed in the acetabulum.

10. The acetabular cup assembly (100) according to any one of claims 2 to 9, wherein said fixing plate (3) comprises:
- a collar (30) shaped like a circumference, disposed around the flat lower edge (12) of the acetabular cup, in such a way to hold the acetabular cup (1) and the head (5) of the prosthesis,
- a plurality of brackets (36, 37, 38, 39) that protrude radially outwards from the collar (30), and
- at least one hole (32) obtained in each one of said brackets in order to accommodate screws (44) suitable for being screwed in the bone of the acetabulum.

## Patentansprüche

1. Hüftgelenkpfannenanordnung (100), umfassend:
- eine Hüftgelenkpfanne (1), die dazu bestimmt ist, in einem Sitz (80) eines Hüftgelenks (8) des Patienten angeordnet zu werden und einen im Wesentlichen kugelförmigen Kopf (5) aufzunehmen, der an einem Schaft (6) befestigt ist, der dazu bestimmt ist, in einem Oberschenkelknochen des Patienten angeordnet zu werden,
- einen Wachstumsträger (2), der dazu bestimmt ist, an der Hüftgelenkpfanne (1) und an dem Pfannensitz (80) befestigt zu werden,
- eine Befestigungsplatte (3), die dazu bestimmt ist, an dem Hüftgelenk (8) des Patienten befestigt zu werden, und
- Befestigungsschrauben (4) zum Befestigen der Hüftgelenkpfanne, des Wachstumsträgers (2) und der Befestigungsplatte (3) am Hüftgelenkpfannenknochen,
wobei
die Hüftgelenkpfanne (1) entsprechend gestaltet ist, um mittels Presspassung mit dem Pfannensitz (80) gekoppelt zu werden,
**dadurch gekennzeichnet, dass**
die Befestigungsplatte (3) ein von der Hüftgelenkpfanne (1) getrenntes Element ist, das dazu geeignet ist, an dem Hüftgelenk (8) befestigt zu werden, um sowohl die Hüftgelenkpfanne (1) als auch den Kopf (5) der Prothese zu halten.

2. Hüftgelenkpfannenanordnung (100), nach Anspruch 1, wobei die Hüftgelenkpfanne (1) die Form einer Kugelkalotte aufweist, umfassend:
- eine konkave Innenfläche (10), die dazu bestimmt ist, den Kopf (5) der Prothese aufzunehmen,
- eine konvexe Außenfläche (11), die dazu bestimmt ist, mit dem Knochen des Patienten im Pfannensitz (80) in Kontakt zu gehen, und
- einen unteren Rand (12) in Form eines flachen Ringes, in dem Löcher (13, 14, 15) zur Aufnahme von Schrauben (4) zur Befestigung der Hüftgelenkpfanne am Hüftgelenk herausgearbeitet sind.

3. Hüftgelenkpfannenanordnung (100) nach Anspruch 2, wobei der untere Rand (12) der Hüftgelenkpfanne durch einen Innenumfang (C1) und einen Außenumfang (C2) definiert ist; wobei der Innenumfang (C1) exzentrisch in Bezug auf den Außenumfang (C2) ist, wobei der Innenumfang (C1) einen Mittelpunkt (O1) aufweist, der von einem Mittelpunkt (O2) des Außenumfangs (C2) beabstandet ist; wobei der untere Rand (12) der Hüftgelenkpfanne einen Abschnitt mit minimaler Dicke (12a) aufweist, der einem Abschnitt mit maximaler Dicke (12b) diametral gegenüberliegt in Bezug auf eine Gerade (r), die durch die Mittelpunkte (O1, O2) des Innenumfangs und des Außenumfangs hindurchgeht.

4. Hüftgelenkpfannenanordnung (100) nach Anspruch 3, wobei die Löcher der Hüftgelenkpfanne ein Zwischenloch (13) umfassen, das peripher im Abschnitt mit maximaler Dicke (12b) des unteren Randes zwischen zwei seitlichen Löchern (14, 15) herausgearbeitet ist, die peripher im unteren Rand (12) der Hüftgelenkpfanne herausgearbeitet sind.

5. Hüftgelenkpfannenanordnung (100) nach Anspruch 4, wobei jedes seitliche Loch (14, 15) vom Zwischenloch (13) um einen Winkel (α) von ungefähr 25 - 35 Grad winklig beabstandet ist.

6. Hüftgelenkpfannenanordnung (100) nach Anspruch 4 oder 5, wobei das Zwischenloch (13) und die seitlichen Löcher (14) im Wesentlichen rechtwinklige Achsen zum unteren Rand (12) aufweisen und auf der konvexen Außenfläche (11) seitlich offen sind, wobei sie sich über eine Höhe von ungefähr 1/5 - 1/2 der Höhe der Hüftgelenkpfanne erstrecken.

7. Hüftgelenkpfannenanordnung (100) nach einem der Ansprüche 2 bis 6, wobei die konvexe Außenfläche (11) der Hüftgelenkpfanne einen Befestigungsabschnitt (16) aufweist, der sich vom unteren Rand (12) über eine Höhe von ungefähr 1/5 - 1/2 der Höhe der Hüftgelenkpfanne erstreckt; wobei der Befestigungsabschnitt (16) dazu geeignet ist, eine Presspassungskopplung mit dem Pfannensitz (80) zu ermöglichen und die Befestigungsplatte (3) in Position zu halten.

8. Hüftgelenkpfannenanordnung (100) nach Anspruch 7, wobei der Befestigungsabschnitt (16) der Hüftgelenkpfanne eine Vielzahl von peripheren Rippen (16a) und peripheren Nuten (16b) zwischen den Rippen aufweist; wobei die peripheren Rippen (16a) die Form eines schraubenförmigen Gewindes und eine abgeschrägte Oberfläche mit nach oben hin abnehmenden Abmessungen aufweisen, derart, dass das Einsetzen der Hüftgelenkpfanne (1) in den Pfannensitz (80) begünstigt wird.

9. Hüftgelenkpfannenanordnung (100) nach einem der Ansprüche 2 bis 8, wobei der Wachstumsträger (2) umfasst:
- eine konkave Innenfläche (21), die einen Querschnitt in Form eines Umfangsbogens aufweist und geeignet ist, mit einem Abschnitt der konvexen Außenfläche (11) der Hüftgelenkpfanne gekoppelt zu werden, derart, dass der Wachstumsträger (2) seitlich aus der Hüftgelenkpfanne vorsteht,
- eine konvexe Außenfläche (22) und
- eine flache, untere Fläche (20) in Form eines Viertelmondes, in der periphere Löcher (23, 24, 25) herausgearbeitet sind, die dazu bestimmt sind, in Übereinstimmung mit den Löchern (13, 14, 15) der Hüftgelenkpfanne angeordnet zu werden, um die Schrauben (40) aufzunehmen, die die durch Löcher der Hüftgelenkpfanne und des Wachtumsträgers hindurchgehen und in das Hüftgelenk eingeschraubt werden;
wobei der Wachstumsträger (2) ein zentrales Loch (26) umfasst, das dazu geeignet ist, eine Schraube (41) aufzunehmen, die dazu bestimmt ist, in das Hüftgelenk eingeschraubt zu werden,

10. Hüftgelenkpfannenanordnung (100) nach einem der Ansprüche von 2 bis 9, wobei die Befestigungsplatte (3) umfasst:
- einen Bund (30), der die Form eines Umfangs aufweist, der um den unteren flachen Rand (12) der Hüftgelenkpfanne angeordnet ist, derart, dass die Hüftgelenkpfanne (1) und der Kopf (5) der Prothese gehalten werden,
- eine Vielzahl von Klammern (36, 37, 38, 39), die aus dem Bund (30) radial nach außen vorstehen, und
- mindestens ein Loch (32), das auf jeder Klammer herausgearbeitet ist, um die Schrauben (44) aufzunehmen, die dazu bestimmt sind, in den Hüftgelenkknochen eingeschraubt zu werden.

## Revendications

1. Groupe prothèse pour acétabule (100) comprenant :
- une cupule (1) destinée à être fixée dans un emplacement (80) d'un acétabulum (8) du patient et à accueillir une tête (5) de forme essentiellement sphérique fixée à une tige (6) apte à être disposée dans un fémur du patient,
- un support de croissance (2) apte à être fixé à la cupule (1) et à l'emplacement acétabulaire (80),
- une plaque de fixation (3) apte à être fixée à l'acétabulum (8) du patient, et
- des vis de fixation (4) pour fixer la cupule, le support de croissance (2) et la plaque de fixation (3) à l'os acétabulaire,
où
la cupule (1) est conformée de manière à pouvoir être couplée en relation press-fit à l'emplacement acétabulaire (80),
**caractérisé en ce que**
la plaque de fixation (3) est un élément séparé de la cupule (1) qui est apte à être fixée à l'acétabulum (8) pour retenir tant la cupule (1) que la tête (5) de la prothèse.

2. Groupe prothèse pour acétabule (100) selon la revendication 1, où la cupule (1) a la forme d'une calotte sphérique comprenant :
- une surface interne concave (10) apte à accueillir la tête (5) de la prothèse,
- une surface externe convexe (11) apte à aller en contact avec l'os du patient dans l'emplacement acétabulaire (80), et
- un bord inférieur (12) de forme annulaire et plat où des orifices (13, 14, 15) sont réalisés pour accueillir des vis (4) pour la fixation de la cupule à l'acétabulum.

3. Groupe prothèse pour acétabule (100) selon la revendication 2, où le bord inférieur (12) de la cupule est défini par une circonférence interne (C1) et par une circonférence externe (C2) ; la circonférence interne (C1) étant excentrique par rapport à la circonférence externe (C2) ; la circonférence interne (C1) ayant un centre (01) écarté d'un centre (02) de la circonférence externe (C2) ; le bord inférieur (12) de la cupule ayant une portion d'épaisseur minimum (12a) diamétralement opposée à une portion d'épaisseur maximum (12b), avec référence à une ligne droite (r) qui passe par les centres (01, 02) de la circonférence interne et de la circonférence externe.

4. Groupe prothèse pour acétabule (100) selon la revendication 3, où lesdits orifices de la cupule comprennent un orifice intermédiaire (13) réalisé en position périphérique dans la portion d'épaisseur maximum (12b) du bord inférieur, disposé entre deux orifices latéraux (14, 15) réalisés en position périphérique dans le bord inférieur (12) de la cupule.

5. Groupe de prothèse pour acétabule (100) selon la revendication 4, où chaque orifice latéral (14, 15) est angulairement espacé de l'orifice intermédiaire (13) d'un angle (α) d'environ 25-35.

6. Groupe prothèse pour acétabule (100) selon la revendication 4 ou 5, où l'orifice intermédiaire (13) et les orifices latéraux (14) ont des axes essentiellement orthogonaux au bord inférieur (12) et sont ouverts latéralement sur la surface externe convexe (11) et se déploient pour une hauteur d'environ 1/5 - 1/2 de la hauteur de la cupule.

7. Groupe prothèse pour acétabule (100) selon l'une quelconque des revendications de 2 à 6, où la surface externe convexe (11) de la cupule a une portion de fixation (16) qui se déploie depuis le bord inférieur (12) pour une hauteur d'environ 1/5 - 1/2 de la hauteur de la cupule ; ladite portion de fixation (16) étant apte à consentir un accouplement de type press-fit avec l'emplacement acétabulaire (80) et bloquer la plaque de fixation (3).

8. Groupe prothèse pour acétabule (100) selon la revendication 7, où la portion de fixation (16) de la cupule a une pluralité de nervures circonférientelles (16a) et de rainures circonférentielles (16b) entre les nervures ; les nervures circonférentielles (16a) ayant la forme d'un filetage hélicoïdal ayant une surface conique avec dimensions décroissantes en allant vers le haut, de manière à favoriser l'insert de la cupule (1) dans l'emplacement acétabulaire (80).

9. Groupe prothèse pour acétabule (100) selon l'une quelconque des revendications de 2 à 8, où ledit support de croissance (2) comprend :
- une surface interne concave (21) ayant en section une forme en arc de circonférence apte à se coupler avec une portion de la surface externe convexe (11) de la cupule de manière à ce que le support de croissance (2) déborde latéralement de la cupule,
- une surface externe convexe (22), et
- une surface inférieure plate (20) en forme d'un quartier de lune, où sont réalisés des orifices périphériques (23, 24, 25) aptes à être disposés en correspondance desdits orifices (13,14, 15) de la cupule pour accueillir des vis (40) qui traversent les orifices de la cupule et du support de croissance et se vissent dans l'acétabulum ;
ledit support de croissance (2) comprenant un orifice central (26) apte à accueillir une vis (41) destinée à être vissée dans l'acétabulum.

10. Groupe prothèse pour acétabule (100) selon l'une quelconque des revendications de 2 à 9, où ladite plaque de fixation (3) comprend :
- un collier (30), ayant la forme d'une circonférence, disposé autour du bord inférieur plat (12) de la cupule, de manière à retenir la cupule (1) et la tête (5) de la prothèse,
- une pluralité de brides (36, 37, 38, 39) qui débordent radialement vers l'extérieur du collier (30), et
- au moins un orifice (32) réalisé sur chacune desdites brides pour accueillir des vis (44) aptes à être vissées dans l'os de l'acétabulum.
